## Europäisches Patentamt
## European Patent Office
## Office européen des brevets

(19)

(11) Publication number: **0 048 247**
**B1**

(12)

# EUROPEAN PATENT SPECIFICATION

(45) Date of publication of patent specification: **11.07.84**

(51) Int. Cl.³: **A 61 F 1/00**

(21) Application number: **81900651.1**

(22) Date of filing: **26.03.81**

(86) International application number:
**PCT/GB81/00059**

(87) International publication number:
**WO 81/02668 01.10.81 Gazette 81/23**

(54) **ORTHOPAEDIC IMPLANTS.**

| | |
|---|---|
| (30) Priority: **27.03.80 GB 8010362** | (73) Proprietor: **NATIONAL RESEARCH DEVELOPMENT CORPORATION**<br>**101 Newington Causeway**<br>**London SE1 6BU (GB)** |
| (43) Date of publication of application:<br>**31.03.82 Bulletin 82/13** | |
| | (72) Inventor: **SCALES, John Tracey**<br>**17 Brockley Avenue**<br>**Stanmore, Middlesex (GB)** |
| (45) Publication of the grant of the patent:<br>**11.07.84 Bulletin 84/28** | |
| | (74) Representative: **Burford, Anthony Frederick et al,**<br>**W.H. Beck, Greener & Co. 7 Stone Buildings**<br>**Lincoln's Inn**<br>**London WC2A 3SZ (GB)** |
| (84) Designated Contracting States:<br>**CH DE FR LI** | |
| (56) References cited:<br>**EP - A - 0 006 544**<br>**DE - A - 2 502 884**<br>**FR - A - 2 216 981**<br>**GB - A - 1 487 181**<br>**GB - A - 1 530 670**<br>**GB - A - 2 004 750**<br>**GB - A - 2 053 687** | |

Courier Press, Leamington Spa, England.

## Description

The present invention relates to orthopaedic implants for the human and animal body and provides such implants which affect bone growth. The invention is concerned in particular, but not exclusively, with an endoprosthetic orthopaedic device having means for promoting bone growth in a selected region proximate to the device.

As used in this Specification the term "orthopaedic implant" means any inanimate article (including a device) which is to be implanted for orthopaedic purposes in the human or animal body for a prolonged period. In particular, the term includes, for example, orthopaedic pins, plates and screws and artificial joints. References to "endoprosthetic implants" include the entire implant, parts thereof and fixing means therefor. Thus, in this Specification fixing pins and screws for use with endoprosthetic devices are included with the term "endoprosthetic implant".

In the art of implanting in the body prostheses for assisting or replacing bone functions such as elbow hip and knee joints, it is the generally established practice to seek to use the most inert materials available for the structural parts of the prostheses so as to avoid undesirable reaction from the body. For example the structural materials used are sought to be free from corrosion when in contact with bodily materials and are also sought to be free from a reaction with bone material which may cause bone reabsorption where the prosthesis is directly attached to bone material or indirectly attached by means of bone cement. Where a component of the prosthesis is attached to bone by a junction which includes for example screws, bolts or force fit, bone reabsorption can produce loosening of the device as the bone recedes away from the attached component. Such direct contract between bone and an implant member may occur where the plate is bolted to strengthen or join or broken bone, or where a component of a prosthetic joint is secured to a neighbouring bone.

It is also general practice when attaching a prosthetic joint such as an elbow joint, for the joint components to be secured by elongate members extending away from the joint, each elongate member being inserted into a cavity reamed in the medulary canal. Before insertion of the prosthetic member, the reamed cavity is filled with a gap filling medium such as a cement, for example a polymethylmethacrylate cement. The member is then pushed into the cement which sets quickly to secure the prosthesis in place. Again however the materials used usually are selected to be as inert as possible and to try to reduce bone reabsorption around the insert.

Those materials used today which most closely approach the ideal of inertness are known as bioaccepptable or bio-passive materials. During the history of development of implant materials, the materials used have progressed from early use of mild steel implants, sometimes secured by mild steel screws and bolts, through the use of surgical stainless steel, to present use of cobalt chromium molybdenum alloys, and titanium alloys. Other materials which are used in prosthetic devices include ceramic and carbon-based materials, and some synthetic plastics materials such as ultra-high molecular weight polythene, some forms of nylon, polymethylmethacrylate, and silicone elastomers. None of these materials have fulfilled entirely the usual ideal of inertness in all circumstances, but in almost all cases the attempt has been towards the use of more fully inert materials to prevent so far as possible any interaction with bodily materials.

It has been proposed to provide in or near an orthopaedic implant a substance to promote bone growth with the specific intention of securing more firmly the implant and/or strengthening the bone in the region of the implant. In particular, French Patent Speification No 2216981 and UK equivalent Patent Specification No 1467069 discloses that bone growth can be stimulated, accelerated, or regulated by diffusion from an alumina ceramic implant of lithium, boron, carbon, fluorine, sodium, magnesium, silicon, phosphorus, potassium and/or calcium ions. The ions are introduced into the surface of the implant by the thermal decomposition of oxides, carbonates or salts. UK Patent Specification No 1487181 discloses a bone replacement material consisting of sintered synthetic apatitic material in which some calcium ions have been replaced by *inter alia* zinc ions to promote wound healing and encourage exotopic bone formation. Reference is also made in UK Patent Specification No 1487181 to the replacement of said calcium ions by magnesium, copper, nickel, cobalt, iron and manganese. European Patent Specification No 006544 discloses the use of completely or partially resorbable calcium phosphate ceramic in and/or on a solid core of tissue-compatible metal as a composite implantable bone substitute material. It is stated that the ceramic reacts with bone cells to fix an implanted prosthesis by bone neoplasm on the implant. Specific reference is made to the use of the composite material as a fixing and screwing member for fastening loaded joint endoprosthesis and in artificial hip joints.

In another branch of medicine concerned with bone material, there may be found in the literature a number of studies concerned with the use of electrical activity to promote bone growth. One example of such a publication is a paper entitled "Treatment of Non-Union with Constant Direct Current" by C. T. Brighton *et al,* Clinical Orthopaedics and Related Research, No. 124 May 1977. Although the phenomena are not entirely understood at present, it appears from the literature that there are a number of relationships between electricity and bone. It is believed that experimental results indicate that stressed bone exhibits electro-negativity in areas of compression, and that living non-stressed bone exhibits electro-negativity in areas of bone growth and healing. Furthermore, it is generally observed that bone growth occurs in areas of compression in the natural workings of the body. It has further been found according to reported experimental evidence that the application of low magnitude direct current to bone induces osteogenesis at the negative electrode or cathode.

Based on the results of work of this nature, there are commercially available devices which seek to promote bone mending and union in cases of fracture where normally bone union does not occur, by the application of electrical activity. Electricity has been reported as being applied to bone by several different techniques, which are referred to in the paper quoted above as including a totally invasive method, in which the electrodes and power pack are implanted in the extremity; a totally non-invasive method in which electromagnetic coils are placed external to the extremity and no part of the apparatus penetrates the skin; and a semi-invasive method in which electrodes penetrate the bone defect and the remainder of the apparatus remains external to the skin. However, two main features of these known treatments are to be noted. Firstly, the use of electricity has been applied for the object of joining together two portions of natural bone material. Secondly, the source of power for the electrical activity has been a man-made power source, and cessation of the electrical activity has been caused by an operator's decision to switch off the power source or by battery failure. Thus these treatments have been essentially externally controlled medical treatments and have had as their object the healing of fractures in natural bone material.

It has been proposed in UK Patent Specification No 2053687 published after the priority date of the present application to use a biogalvanic couple to promote bone growth. In particular, a silver electrode is connected via a resistor to a platinum electrode having a minute quantity of magnesium at its free end. The silver electrode extends through the medullary canals of a bone graft and of adjacent portions of bone sections to be joined by the graft. The magnesium forms a strong galvanic couple with the silver electrode initially driving the silver electrode positive as an anode and thereby producing a germicidal effect. The magnesium is consumed in a few days and thereafter the silver electrode and platinum electrode create a biogalvanic couple with body material which drives the silver electrode as a cathode.

Another area of known phenomena in relation to bone growth concerns observations which have been made and have been published in relation to corrosion in early forms of prosthetic implants where the materials used were not bio-acceptable in the sense in which the term has come to be accepted in modern practice. For example in several known cases where mild steel implants were secured in place by screws of dissimilar material such as iron screws, it has been observed that massive corrosion took place within the body, and this was accompanied by considerable promotion of bone growth. The end result of such early implants was disintegration of the prosthetic implant member, which failed to retain its structural integrity and so failed to carry out its function, together with continuous uncontrolled bone growth which it is believed eventually resulted in the bone dying through the cutting off of the blood supply to the living bone cells. Only at this stage of dying of the bone material did the bone growth cease. However, the corrosion and disintegration of the implant continued until failure occurred.

The present invention has a number of objects, and wide applications in different areas concerned with medical activities on and relating to bone. The objects are differently attained in different aspects of the invention, and in some senses relate to different preferred arrangements of the present invention.

It is one object of the present invention to provide an implant for promotion of bone growth in chosen regions of bone material, and such implant finds application in various treatments of bone.

It is another object of the present invention to provide an improved endoprosthetic orthopaedic device having means for promoting bone growth in selected regions proximate to the endoprosthetic device.

In accordance with the invention there is provided an orthopaedic implant comprising a biologically inactive bio-acceptable structural component for implanting in, on or near bone material in the body, and a bioactive metallic control component for implanting in, on or near said bone material to promote bone growth in the region of said structural component, characterised in that said components serve as electrodes interacting *in situ* with bodily material to form an electric cell with said control component constituting a cathode producing an area of electric negativity to promote bone growth in the region of said structural component and in that said control component being bio-degradable to limit the duration of the said promotion of bone growth.

The control component can be discrete from, attached to or integral with the structural component.

The structural component can serve merely as a substrate for the control component but preferably constitutes an endoprosthetic implant.

According to one preferred embodiment of the apparatus aspect of the present invention, there is provided an endoprosthetic orthopaedic device including an endoprosthetic component of biologically inactive, bio-acceptable material for insertion in or otherwise attaching to bone material in the body, and a metallic control component for implanting in or in the region of the said bone material in co-operation with the said endoprosthetic component, said components serving as electrodes interacting *in situ* with bodily material to form an electric cell with said control component constituting a cathode producing an area of electric negativity to promote bone growth in the region of the endoprosthetic component, and said control component consisting of or including bio-active material which is bio-degradable to limit the duration of the said promotion of bone growth.

In a preferred arrangement of the aforementioned embodiment of the invention, the endoprosthetic component is an elongate member for insertion in a cavity in a bone, and the control component is in the form of a hollow cap or ring dapted to receive the end of the elongate member. Conveniently, the endoprosthetic component is made of titanium and/or titanium alloy and the control component is made of surgical steel carrying a layer of brazing or silver solder.

The quantity of bio-active material provided in the implant device may be chosen so as to predetermine the duration of the promotion of bone growth, until the bio-degradation of the bio-active material brings about substantial cessation of the bone growth. Where reference is made in this specification to selection of the bio-active material (in relation to other materials present) so as to predetermine the duration of bio-activity, it is to be appreciated that such predetermination of a time interval is necessarily approximate, and may be in the region of for example two or three years.

It will be appreciated that in the first aspect of the invention as set out above, the endoprosthetic component and the control component may be assembled either before, during or after the insertion of the components in the body. For example the assembly may be put together by firstly inserting in a cavity in bone material the control component, followed by insertion of the endoprosthetic component into the cavity.

Closely related to the invention as set out in the first aspect above, there may be provided in accordance with a second aspect of the invention a component for an endoprosthetic orthopaedic device, the endoprosthetic component being adapted for insertion in or otherwise attaching to bone material in the body and having a composite form comprising biologically inactive, bio-acceptable material and bio-active metallic material, said materials serving as electrodes interacting *in situ* with bodily material to form an electric cell with said bio-active material constituting a cathode producing an area of electric negativity to promote bone growth in the region of the endoprosthetic component, and said bio-active material being bio-degradable to limit the duration of the said promotion of bone growth.

In this embodiment of the invention, the bio-active material may conveniently be provided for example by a layer of bio-active material formed directly on part of the said member of biologically inactive material. For example the inactive member may comprise an elongate member of a prosthesis intended to be secured in a cavity reamed in a bone, and the bio-active material may take the form of a layer of brazing formed on the end of the elongate member. Alternatively, bio-active material may be deposited by plating, or by ion deposition, or by impaction (for example D gun coating).

Thus in accordance with the two embodiments of the invention set out above, two forms of implant apparatus have been set out for use in connection with an endoprosthetic orthopaedic device. In accordance with a further embodiment, there may be provided an endoprosthetic orthopaedic device when the endoprosthetic orthopaedic device is inserted in situ in the body. Thus in accordance with this further embodiment there is provided an endoprosthetic orthopaedic device when inserted in the body, the device including an elongate member of biologically inactive, bio-acceptable material inserted in bone material in the body, and bio-active metallic material in the region of the inserted end of the elongate member, said materials serving as electrodes interacting *in situ* with bodily material to form an electric cell with said bio-active material constituting a cathode producing an area of electric negativity to promote bone growth in the region of the inserted end of said elongate member and said bio-active material being bio-degradable to limit the duration of the said promotion of bone growth.

In accordance with this embodiment of the invention, the invention provides particular advantage in that the siting of the material in the region of the inserted end of the elongate member can be arranged to produce bone growth at a region of the bone which is particularly susceptible to breakage after the insertion of the distal end of an endoprosthetic component.

The endoprosthetic orthopaedic device may conveniently be made of titanium, or titanium alloy, or cobalt chrome molybdenum alloy, or ceramic material, or synthetic plastics material, or any combination of the materials.

In connection with a fourth general embodiment of the apparatus aspect of the invention, there may be provided an implant structure for assisting or replacing mechanical bone function in the body comprising a functional structural member of biologically inactive bio-acceptable material for insertion in or otherwise attaching to bone material in the body, and a bio-active metallic material, said materials serving as electrodes interacting *in situ* with bodily material material to form an electric cell with said bio-active material constituting a cathode producing an area of electric negativity to promote bone growth in the region of the inactive member, the bio-active material being bio-degradable to lim' the said promotion of bone growth, and the said structural member being such as to retain its mechanical integrity and to be bio-acceptable in the body after the bio-degrading of the active material.

In this fourth embodiment, the said functional structural member may for example consist of a plate fastened to a bone across a fracture and intended to strengthen the bone after healing has taken place. One feature of the invention in this embodiment is that the biologically inactive component is a functional structural member, e.g. having a purpose in the body after the bio-degrading of the bio-active material, and it is a feature that the structural member retains its mechanical integrity after the bio-degrading of the active material.

In a fifth embodiment of the invention there may be provided a control device for control of bone growth, the control device being adapted for implanting in the body in or in the region of bone material, and having a composite form comprising biologically inactive, bio-acceptable material, and bio-active metallic material, said materials constituting electrodes interacting *in situ* with bodily material to form an electric cell with said bio-active material constituting a cathode producing an area of electric negativity to promote bone growth, the bio-active material being bio-degradable to limit the duration of the said promotion of bone growth and in such a manner as to leave the control device in a bio-acceptable condition to remain in the body after the bio-degrading of the active material.

It is to be appreciated of course that any particular implant in accordance with the invention may fall within more than one embodiment as set out above, and the various aspects have been delineated merely in order to emphasise various features of the inventive concept.

In the preceding paragraphs the apparatus aspects of the invention have been set out with regard to an endoprosthetic orthopaedic device, a component of an endoprosthetic orthopaedic device, an endoprosthetic orthopaedic device when inserted in the body, an implant structure and a control device. In this specification these items will be referred to by the general term apparatus when considering further features of the invention.

Considering other terms which have been used in connection with the invention, the term biologically inactive, bio-acceptable material means a material which is normally acceptable to the body as an implant material and does not of itself interact with normal bodily materials. It is to be appreciated however that such material interacts with bodily materials when in combination with the said bio-active material to constitute the anode of the electric cell.

By a bio-active material is meant a material which either by itself, or when in combination with the said biologically inactive material, will normally interact electrically and/or chemically with bodily materials when implanted in the body. By the term bio-degrading is meant a change in the chemical structure of a material by interaction with bodily material when the material is implanted in the body. For example the bio-degrading may take the form of dissolving the bio-active material to limit the bone promotion, or alternatively the bio-degrading may take the form of a build-up of corrosion products on the surface.

There will now be set out a number of preferred features of the invention which in general may be applicable in the various embodiments where appropriate, as set out above.

As has been noted above, the said bio-active material is to promote bone growth as a result of a combined interaction between the bio-active material and the said inactive material when in contact with bodily materials when implanted in the body. The said two materials are selected to be such that in use when implanted in the body the bio-active material acts as a sacrificial cathode producing an area of electrical negativity to promote bone growth.

The said bio-degradation of the bio-active material may take a number of forms. For example the bio-degraded material may be secreted by the body as a waste product, or may be distributed around the body and stored in a non-harmful form.

Although as has been mentioned, a number of different materials may be used in accordance with the invention, it is preferred that the said biologically inactive material consists of titanium and/or titanium alloy and/or cobalt chromium molybdenum alloy. For example the titanium alloy may comprise titanium alloy Type 318, which is an alloy of 6% aluminium and 4% vanadium.

The said bio-active material may conveniently include or consist of one or more of the elements comprising iron, copper, tin, zinc or silver, and may conveniently consist of a layer of brazing or silver solder, for example on surgical stainless steel.

It will be appreciated that the capability in accordance with the invention of producing bone growth allows many uses in medical treatments.

In an application of the implant of the present invention, the bio-active material may be located in a position such as to promote bone growth in the region of the inserted end of an elongate member of an endoprosthetic orthopaedic device where said end is inserted in bone material. As has been mentioned this region at the end of an inserted endoprosthetic component is particularly vulnerable to breakage, and a thickening of bone at this region is particularly advantageous. In addition, or as an alternative application, the invention may be used to promote bone growth around an endoprosthetic component so as to secure the component more safely in the bone material.

It will be appreciated that the present invention differs significantly from the previously known art in relation to bone treatment. In particular, it differs from present practice in the choice of materials for endoprosthetic devices in that a deliberate choice is made of bio-active material to be included in the endoprosthetic device, whereas present practice tends to the selection of materials which are to the greatest extent possible bio-passive and bio-acceptable. Further, it differs from previous proposals to promote bone growth in the region of an orthopaedic implant by the use of bio-active substances in that it makes use of a galvanic couple established for a limited time between the bio-active material as sacrificial cathode and the implant. Accordingly, the present invention is of particular application to metallic implants.

With regard to the commercially available devices which promote bone growth by application of electrical activity, it will be seen that the present invention is distinguished by virtue of the fact that the power source for the electrical activity is provided by the bio-active material itself situated in the body. The prior art is also distinguished in this aspect in that the termination of the electrical activity is not required to be triggered by an external switching-off of the power source, but arises naturally by the bio-degradation of the bio-active material.

With regard to the proposal to use a biogalvanic couple to promote bone growth, the present invention is distinguished in that it is concerned with orthopaedic implants and not bone grafts and, more significantly, the implant constitutes the anode of the electric cell whilst retaining its long term biologically inactive bio-acceptable nature.

With regard to the known observations in relation to early endoprosthetic devices which included mild steel and other bio-active materials, it is to be noted that in accordance with the present invention there is included biologically inactive material which is intended to remain in the body (e.g. as a permanent implant) after cessation of the bio-activity and bio-degradation. In those observations of the early endoprosthetic devices where bone growth occurred during bio-activity of the inserted material, it is to be noted that the end result was an ineffective faulty endoprosthetic device which deteriorated by massive corrosion until it could no longer perform its required function. In various embodiments there is provided a member of biologically inactive material which retains its mechanical integrity after the cessation of bio-activity.

Embodiments of the invention will now be described by way of example with reference to the accompanying drawings in which:—

Figure 1 is a diagrammatic representation of a section through the distal end of an endoprosthetic component;

Figure 1a is a diagrammatic cross-section through the end of an endoprosthetic component showing an optional feature for securing the component;

Figure 1b shows a modification of the component of Figure 1;

Figure 2 is a drawing taken from an X-ray of an endoprosthetic orthopaedic device comprising an elbow joint in situ at the time of implanting; and

Figure 3 is a drawing taken from an X-ray of the same endoprosthetic device at a time approximately five years after the implantation.

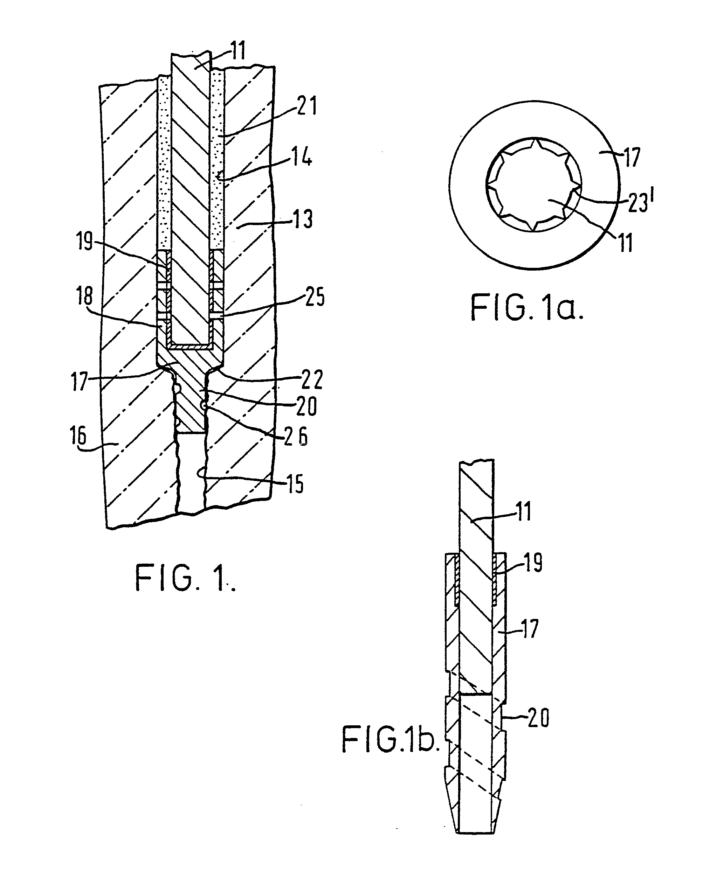

Referring firstly to Figure 1, a member 11 comprises an elongate member of an endoprosthetic device 13 consisting of an elbow joint. The member 11 is situated in a reamed out cavity 14 in the medulary canal 15 of a forearm bone 16.

At the distal end of the member 11 is positioned a control component comprising an implanted element 17 in the form of a cap. The element 17 comprises a main body 18 having on its interior surface a layer 19 of bio-active material such as will interact with bodily material to give an effect as will be described hereinafter to promote bone growth. As has been mentioned, the main body 18 is in the shape of a cap co-operating with the end of the member 11, and has a threaded extension 20 which protrudes into the unreamed medulary canal 15. The space between the member 11 and the reamed cavity 14 is filled by a cap filling medium 26 such as bone cement.

Considering the materials from which the various components may be made, the endoprosthetic member 11 may conveniently be made of titanium alloy type 318, and the bone cement 26 may be conventional poly methyl methacrylate. The main body 18 of the cap element 17 may be formed of the same titanium alloy 318, or may be formed of surgical stainless steel, or cobalt chromium molybdenum alloy. The layer 19 of bio-active material may comprise a layer of brazing having a composition of, for example, 60% copper, 35% tin, with trace elements of manganese silicon and nickel, and the remainder of the composition zinc. As an alternative, the layer 19 may comprise silver solder of a commonly available commercial composition having a basic formula of 50% silver with the remainder composed of copper and zinc.

Considering the dimensions and configuration of the cap element 17, the element may be for example $2\frac{1}{2}$ centimetres long and 4 millimetres in diameter at its upper open end. The layer 19 of bio-active material may extend around the entire interior surface of the element 17 as shown, or alternatively the layer may comprise a layer of brazing or silver solder which extends for only one-half centimetre down the main body 18 from the open end thereof (see Figure 1b). In another modification there may be provided perforations 25 in the hollow cap part of the main body 18 extending through to the interior of the cap. The extension 20 is conveniently either a force fit in the medulary canal 15, or is threaded so as to be secured to the interior surface of the canal.

There will now be described the method of insertion in the bone 16 of the assembly of the implant 17 and endoprosthetic member 11. Firstly the medulary canal 15 is reamed out in conventional manner by a surgical reamer to a depth sufficient to accept the member 11, and slightly oversize of the member 11, terminating in a step 22. There are then a number of ways of assembling the components. In a first method the element 17 is lodged on the end of an inserting rod (not shown) and is pushed down the reamed cavity 14 until the extension 20 lodges in the medulary canal 15. In a preferred arrangement (not shown) the inserting rod co-operates with flanges on the interior of the element 17 so that the inserting rod can be rotated and threads on the extension 20 can be engaged with the interior of the medulary canal 15. The inserting rod is so arranged that a slight reverse turn releases the inserting rod from the element 17 and the inserting rod can be withdrawn leaving the element 17 in place. Next the reamed cavity 14 is filled by conventional means with a bone cement 21 and finally the member 11 is pushed through the bone cement down into the cavity 14 so as to lodge in the element 17. It is thought to be advantageous for the member 11 to have a number of points of contact, if not a complete area of contact, with the interior of the element 17, and to this effect the exterior of the end of the member 11 may be formed with outwardly-extending peaks or serrations indicated at 23' in Figure 1a. These protrusions 23' ensure good contact with the element 17. In other arrangements the interior of the element 17 may have internally-projecting ribs or protrusions which effect the same connection with a smooth ended member 11. It will be appreciated that some bone cement 21 will be carried into the interior of the element 17 by the member 11, but it is generally found that sufficient contact is made between the member 11 and the element 17 by the member 11 being forced into the element 17.

It is to be appreciated that a number of variations may be made in this arrangement. For example where the member 11 is substantially straight, it is possible to affix the cap element 17 directly onto the member 11 before insertion in the bone, and the securing of the element 17 can be effected by rotating the entire member 11 so as to screw the extension 20 into the medulary canal 15. In another variation, the layer of brazing or silver solder 19 may be applied directly to the end of the member 11, and the element 17 may be a plain titanium alloy.

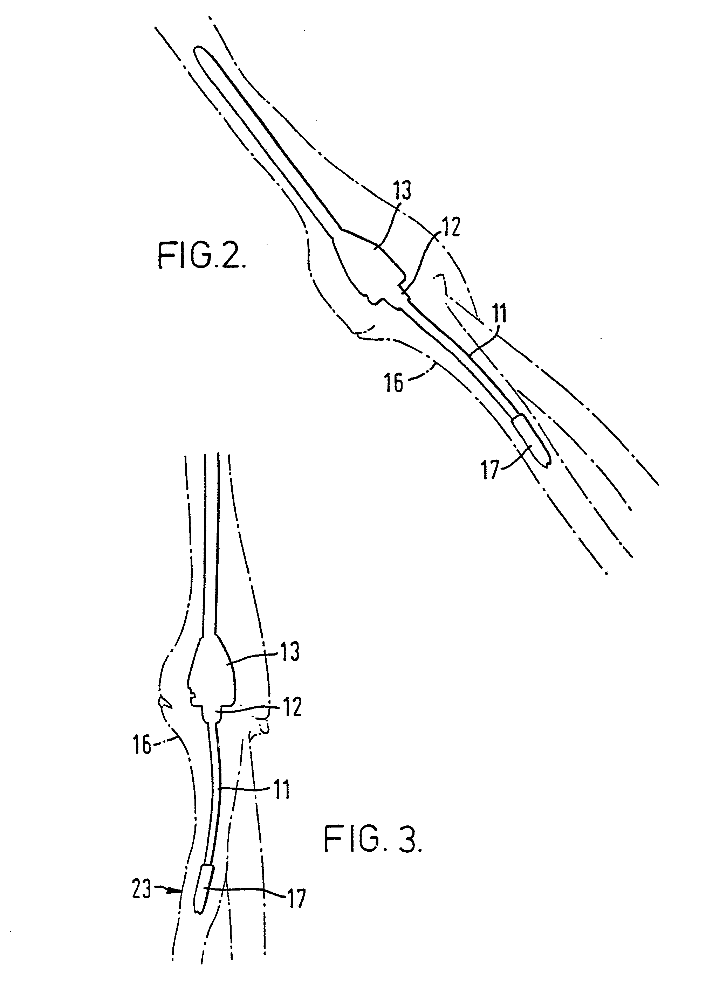

Referring now to Figures 2 and 3, there will be described a promotion of bone growth which has been observed where the element 17 consists of a main body 18 of surgical cutting steel to British Standard EN 56D having a layer of silver solder or brazing to a depth of one-half centimetre along a 2 centimetre long implant (as shown in Figure 1b), and having a diameter of 4 millimetres. The member 11 was inserted as a force fit in the interior of the element 17, and in this example no perforations were provided through the element 17. The element 17 was threaded as shown in Figure 1b and had been firmly screwed into the medulary canal 15.

Figure 2 is a drawing taken from an X-ray taken at the time of implant in the patient, and Figure 3 is a drawing taken from an X-ray taken of the same insert approximately five years later. As is shown in Figure 3 there had occurred a thickening of the bone indicated at 23 and appearing in the immediate region of the element 17. It will be appreciated that the thickening of bone in this region at the distal end of a prosthetic component is particularly advantageous since it is at this region that the bone is particularly susceptible to breakage due to the stiffening effect of the endoprosthetic member in the bone. The bone is particularly susceptible to breaking where this stiffening effect terminates, and the bone thickening shown in Figure 3 occurs at this region which is normally a region of weakness.

There will now be given what is believed to be an explanation of the activities involved in the production of the bone promotion described, although it is to be appreciated that the operation of the invention does not necessarily depend upon the accuracy of the following explanation. It is believed that when the assembly of the member 11 and element 17 are inserted into the bone 16 in intimate contact with the bone and other bodily material, an electric cell is set up between the metallic components provided, in which the layer of brazing or silver solder 19 constitutes a sacrificial cathode. Over a limited period of time, in the region of 2—3 years, it is believed that an area of electrical negativity is provided at the element 17 which has the effect of promoting bone growth around the element. During this activity, it is believed that the layer 19 constitutes bio-active material which biodegrades by electro-chemical action and dissolves into ions which are non-harmful to the body and which are distributed by bodily fluids away from the implant, either to be stored by the body in non-harmful manner, or to be secreted from the body, or to be dissipated by a combination of these effects. It is believed that the effect is dependent upon, or enhanced by contact between the element 17 and the bone structure, and by contact between the member 11 and the element 17. It is also believed that in other arrangements, the effect may be enhanced by perforations through the element 17 allowing a greater area of contact of bodily materials with both the main body 18 and the layer 19 of bio-active material.

There will now be described a number of alternative constructions of apparatus embodying the invention, and various applications of the apparatus.

In some arrangements it may be advantageous to arrange for all the elements of the assembly to be provided upon a single member, by electroplating or otherwise coating the main member with different metals to produce the required electrical effect. In another arrangement the separate implant elements may consist of one or more bands or rings of metal such as plain iron, inserted into the bone at a distance spaced from the main structural member.

It is believed that the current is in the range of 20 to 120 microamps.

There will now be described a number of applications of the present invention, and in these general descriptions, the invention will be referred to in its general terms as involving the use of an implant element of biologically inactive bio-acceptable material, and bio-active material such as will interact with bodily material to promote bone growth. Clearly one primary application of the invention is in the production of bone growth at the distal end of an endoprosthetic orthopaedic component, as has been described. Following this, another application of the invention lies in controlled promotion of bone growth in the region of a repair plate screwed or otherwise secured to a fractured bone across the fracture to strengthen the bone during healing.

It will be appreciated that another important effect of the bone thickening shown in Figure 3 is that the distal end of the endoprosthetic insert is more securely fastened in the bone by the bone growth which knits around the insert. Thus in other embodiments there may be provided endoprosthetic components inserted in bone in which bio-active material is so sited as to encourage bone to grow into and around the prosthetic components so as to provide a natural locking of the prosthetic components into the bone. In some developments of this application of the invention it may be possible to secure endoprosthetic components in bone without the use of bone cement as a gap filling agent.

**Claims**

1. An orthopaedic implant comprising a biologically inactive bio-acceptable structural component for implanting in, on or near bone material in the body, and a bioactive metallic control component for implanting in, on or near said bone material to promote bone growth in the region of said structural component, characterised in that said components (11, 19) serve as electrodes interacting *in situ* with bodily material to form an electric cell with said control component (19) constituting a cathode producing an area of electric negativity to promote bone growth in the region of said structural component (11) and in that said control component (19) is bio-degradable to limit the duration of the said promotion of bone growth.

2. An orthopaedic implant as claimed in Claim 1 which is an endoprosthetic orthopaedic device, the structural component being an endoprosthetic component.

3. An orthopaedic implant as claimed in Claim 1 which is a component for an endoprosthetic orthopaedic device, said endoprosthetic component having a composite form comprising biologically inactive, bio-acceptable material constituting the structural component and bio-active metallic material constituting the control component.

4. An orthopaedic implant as claimed in Claim 1 which is an endoprosthetic orthopaedic device when inserted in the body, the structural component being an elongate member inserted in bone material in the body, and the control component being in the region of the inserted end of the elongate member.

5. An orthopaedic implant as claimed in Claim 1 which is an implant structure for assisting or replacing mechanical bone function in the body, the structural component being a functional structural member which retains its mechanical integrity and is bio-acceptable in the body after the bio-degrading of the control component.

6. An orthopaedic implant as claimed in Claim 1 which is a control device for control of bone growth, the control device being left in a bio-acceptable condition to remain in the body after the bio-degrading of the active material.

7. An orthopaedic implant as claimed in Claim 2 wherein the endoprosthetic component is an elongate member for insertion in a cavity in a bone, and the control component is in the form of a hollow cap or ring adapted to receive the end of the elongate member.

8. An endoprosthetic orthopaedic device as claimed in Claim 2 or Claim 3 wherein the endoprosthetic component is made of titanium and/or titanium alloy and the control component is made of surgical steel carrying a layer of brazing or silver solder.

9. An orthopaedic implant as claimed in any one of the preceding Claims wherein the said biologically inactive bio-acceptable material is metallic.

10. An orthopaedic implant as claimed in any one of the preceding Claims wherein the said biologically inactive material consists of titanium and/or titanium alloy and/or cobalt chrome molybdenum alloy.

11. An orthopaedic implant as claimed in any one of the preceding Claims wherein the bio-active material includes or consists of one or more of iron, copper, tin, zinc and silver.

12. An orthopaedic implant as claimed in Claim 11 wherein the said bio-active material consists of a layer of brazing or silver solder.

13. An orthopaedic implant as claimed in any one of the preceding Claims wherein the bio-active material is located in a position such as to promote bone growth in the region of the inserted end of an elongate member of an endoprosthetic orthopaedic device when said end is inserted in bone material.

## Revendications

1. Implant orthopédique comprenant un composant structural bio-acceptable et biologiquement inactif pour une implantation dans, sur ou à proximité d'un matière osseuse dans le corps, et un composant de commande métallique bio-actif pour une implantation dans, sur ou à proximité de ladite matière osseuse afin de promouvoir une croissance osseuse dans la zone dudit composant structural, caractérisé en ce que lesdits composants (11, 19) servent d'électrodes réagissant *in situ* avec la matière osseuse pour former une pile électrique, ledit composant de commande (19) constituant une cathode produisant une zone de comportement électrique négatif pour favoriser la croissance osseuse dans la région dudit composant structural (11) et en ce que ledit composant de commande (19) est biodégradable pour limiter la durée de ladite promotion de croissance osseuse.

2. Implant orthopédique comme revendiqué dans la revendication 1, qui est un dispositif orthopédique d'endoprothèse, le composant structural étant un composant d'endoprothèse.

3. Implant orthopédique comme revendiqué dans la revendication 1, qui est un composant pour un dispositif orthopédique d'endoprothèse, ledit composant d'endoprothèse ayant une forme composite comprenant une matière bio-acceptable et biologiquement inactive constituant le composant structural et une matière métallique bio-active constituant le composant de commande.

4. Implant orthopédique comme revendiqué dans la revendication 1, qui est un dispositif orthopédique d'endoprothèse lorsqu'il est inséré dans le corps, le composant structural étant un élément allongé inséré dans une matière osseuse du corps et le composant de commande étant placé dans la région de l'extrémité insérée de l'élément allongé.

5. Implant orthopédique comme revendiqué dans la revendication 1, qui est une structure d'implant pour assister ou remplacer une fonction mécanique d'os dans le corps, le composant structural étant un élément structural fonctionnel qui conserve son intégrité mécanique et qui est bio-acceptable dans le corps après la bio-dégradation du composant de commande.

6. Implant orthopédique comme revendiqué dans la revendication 1, qui est un dispositif de commande pour commander une croissance d'os, le dispositif de commande étant laissé dans une condition bio-acceptable pour rester dans le corps après la bio-dégradation de la matière active.

7. Implant orthopédique comme revendiqué dans la revendication 2, dans lequel le composant d'endoprothèse est un élément allongé destiné à être inséré dans une cavité d'un os et l'élément de commande se présente sous forme d'un chapeau creux ou d'un anneau agencé pour recevoir l'extrémité de l'élément allongé.

8. Dispositif orthopédique d'endoprothèse comme revendiqué dans la revendication 2 ou la revendication 3, dans lequel le composant d'endoprotèse est formé de titane et/ou d'un alliage de titane, et le composant de commande est formé d'acier chirurgical portant une couche de matière de brasage ou de brasure d'argent.

9. Implant orthopédique comme revendiqué dans l'une quelconque des revendications précédentes, dans lequel ladite matière bio-acceptable et biologiquement inactive est métallique.

10. Implant orthopédique comme revendiqué dans l'une quelconque des revendications précédentes, dans lequel ladite matière biologiquement inactive se compose de titane et/ou d'un alliage de titane et/ou d'un alliage de cobalt chrome-molybdène.

11. Implant orthopédique comme revendiqué dans l'une quelconque des revendications précédentes, dans lequel la matière bio-active comprend ou se compose d'un ou plusieurs des éléments suivants: fer, cuivre, étain, zinc, argent.

12. Implant orthopédique comme revendiqué dans la revendication 11, dans lequel ladite matière bio-active se compose d'une couche de matière de brasage ou de brasure d'argent.

13. Implant orthopédique comme revendiqué dans l'une quelconque des revendications précédentes, dans lequel la matière bio-active est placée dans une position telle qu'elle favorise une croissance osseuse dans la région de l'extrémité insérée d'un élément allongé d'un dispositif orthopédique d'endoprothèse lorsque ladite extrémité est insérée dans une matière osseuse.

## Patentansprüche

1. Orthopädisches Implantat, mit einem biologisch inaktiven, körperverträglichen Bauteil zur Implantation in oder nahe von Knochenmaterial in dem Körper, und mit einem bioaktiven metallischen Steuerungsteil zur Implantation in das oder nahe des Knochenmaterials zur Verbesserung des Knochenwaschstums in dem Bereich des Bauteils, dadurch gekennzeichnet, daß die Teil (11, 19) als Elektroden dienen, die in situ zusammenwirken mit Körpermaterial, um ein elektrisches Element zu bilden, wobei das Steuerungsteil (19) eine Kathode bildet, die ein Feld aus elektrischer Negativität erzeugt, um das Knochenwachstum in dem Bereich des Bauteils (11) zu fördern, und daß das Steuerungsteil (19) biologisch abbaubar ist, um die Dauer der Verbesserung des Knochenwachstums zu begrenzen.

2. Orthopädisches Implantat nach Anspruch 1, dadurch gekennzeichnet, daß es eine orthopädische endoprothetische Vorrichtung ist und daß das Bauteil ein endoprothetisches Teil ist.

3. Orthopädisches Implantat nach Anspruch 1, dadurch gekennzeichnet, daß es ein Teil für eine orthopädische endoprothetische Vorrichtung ist, und daß das endoprothetische Teil eine zusammengesetzt Form aufweist aus einem biologisch inaktiven, körperverträglichen Material, das das Bauteil bildet, und aus bioaktivem metallischem Material, das das Steuerungsteil bildet.

4. Orthopädisches Implantat nach Anspruch 1, dadurch gekennzeichnet, daß es eingesetzt in den Körper eine orthopädische, endoprothetische Vorrichtung ist, daß das Bauteil ein langgestrecktes Teil ist, welches in das Knochenmaterial in dem Körper eingesetzt ist, und daß das Steuerungsteil im Bereich des eingesetzten Endes des langgestreckten Teiles angeordnet ist.

5. Orthopädisches Implantat nach Anspruch 1, dadurch gekennzeichnet, daß es einen Implantataufbau aufweist zur Unterstützung oder zum Ersatz mechanischer Knochenfunktion in dem Körper, und daß das Bauteil ein funktionales Bauteil ist, welches seine mechnische Integrität zurückbehält und in dem Körper nach dem biologischen Abbau des Steuerungsteils körperverträglich ist.

6. Orthopädisches Implantat nach Anspruch 1, dadurch gekennzeichnet, daß es eine Steuerungsvorrichtung ist zur Steuerung des Knochenwachstums, und daß die Steuerungsvorrichtung in einem körperverträglichen Zustand belassen wird, um in dem Körper nach dem biologischen Abbau des aktiven Materials zu verbleiben.

7. Orthopädisches Implantat nach Anspruch 2, dadurch gekennzeichnet, daß das endoprothetische Teil ein langgestrecktes Teil ist zum Einsetzen in einem Hohlraum in einem Knochen, und daß das Steuerungsteil in Form einer hohlen Kappe oder eines Ringes ausgebildet ist, um das Ende des langgestreckten Teils aufzunehmen.

8. Orthopädische, endoprothetische Vorrichtung nach Anspruch 2 oder 3, dadurch gekennzeichnet, daß das endoprothetische Teil aus Titan und/oder einer Titanlegierung und das Steuerungsteil aus einem chirurgischen Stahl mit einer Schicht aus Messing-oder Silberlot besteht.

9. Orthopädisches Implantat nach einem der vorhergehenden Ansprüche, dadurch gekennzeichnet, daß das biologisch inaktive körperverträgliche Material Metall ist.

10. Orthopädisches Implantat nach einem der vorhergehenden Ansprüche, dadurch gekennzeichnet, daß das biologische inaktive Material aus Titan und/oder einer Titanlegierung und/oder einer Kobaltchrommolybdän-Legierung besteht.

11. Orthopädisches Implantat nach einem der vorhergehenden Ansprüche, dadurch gekennzeichnet, daß das bioaktive Material aus einem oder mehreren der Elemente Eisen, Kupfer, Zinn, Zink und Silber besteht oder eines oder mehrere dieser Elemente aufweist.

12. Orthopädisches Implantat nach Anspruch 11, dadurch gekennzeichnet, daß das bioaktive Material aus einer Schicht aus Messing-oder Silberlot besteht.

13. Orthopädisches Implantat nach einem der vorhergehenden Ansprüche, dadurch gekennzeichnet, daß das bioaktive Material derart angeordnet ist, daß es das Konochenwachstum in dem Bereich des eingesetzten Endes eines langgestreckten Teiles einer orthopädischen endoprothetischen Vorrichtung fördert, wenn das Ende in das Knochenmaterial eingesetzt ist.

FIG. 1.

FIG.1a.

FIG.1b.

FIG.2.

FIG. 3.